# EUROPEAN PATENT SPECIFICATION

(11) **EP 4 061 323 B1**
(45) Date of publication and mention of the grant of the patent: **20.12.2023**
(21) Application number: 20824639.7
(22) Date of filing: 19.11.2020
(51) Int. Cl.: A61K 8/60, A61K 8/9789, A61Q 5/06

(54) **COLOURING COMPOSITION BASED ON DYE PLANTS AND USE THEREOF FOR HAIR DYEING**
FÄRBEZUSAMMENSETZUNG BASIEREND AUF FÄRBEPFLANZEN UND VERWENDUNG DAVON ZUM HAAREFÄRBEN
COMPOSITION COLORANTE À BASE DE PLANTES COLORANTES ET SON UTILISATION POUR LA TEINTURE DES CHEVEUX

(30) Priority: 20.11.2019 IT 201900021720
(43) Date of publication of application: 28.09.2022
(73) Proprietor: Giuliani S.p.A., 20129 Milano (IT)
(72) Inventor: GIULIANI, Giammaria, 6926 Montagnola (CH); MARZANI, Barbara, 27020 Carbonara Al Ticino (IT); MASCOLO, Antonio, 20126 MILANO (IT)
(74) Representative: Cattaneo, Elisabetta
(86) International application number: PCT/IB2020/060884
(87) International publication number: WO 2021/099974

(56) References cited:
- CN-A- 107 049 873
- US-A- 5 635 461
- Wilma F Bergfeld ET AL: "Safety Assessment of Saccharide Esters as Used in Cosmetics The 2016 Cosmetic Ingredient Review Expert Panel members are: Chair", , 13 January 2017 (2017-01-13), page 88, XP055720235, Retrieved from the Internet: URL:https://www.cir-safety.org/sites/defau lt/files/SACEST122016rep.pdf [retrieved on 2020-08-04]

## Description

### FIELD OF THE INVENTION

The invention relates to a colouring composition based on dye plants and use thereof for hair dyeing.

The present invention relates to the cosmetic sector of hair colouring with products of natural origin, in particular powdery colouring products.

Specifically, the invention relates to compositions for the extemporaneous colouring of hair containing colouring agents of vegetable origin in the form of powder or granules and a suitable non-ionic dispersing/wetting surfactant which facilitates the uniform dispersion of the colouring powder on the hair fibre.

### STATE OF THE ART

Various methods for hair colouring are known in the cosmetic field. The classification of the colouring methods may vary and typically reflects the type of process underlying the colouring technique (oxidative, non-oxidative or direct), the duration of the colour after application following washes (temporary, semi-permanent, permanent), the time in which the colouring develops (instantaneous, progressive) or the nature of the colouring (with metal salts, with dye plants) etc...

The following colouring techniques can therefore be distinguished: temporary or transient colouring; semi-permanent, also called direct, colouring; demipermanent colouring; permanent colouring; colouring with dye plants; colouring with metal salts. Temporary colouring temporarily changes the colour of the hair thanks to the use of ready-to-use products that deposit large-sized colouring substances on the hair that remain on the cuticular surface just for one or two washes.

Permanent colouring envisages the use of oxidation bases which, combined with suitable couplers, in an alkaline environment following the oxidation process, give rise to coloured molecules.

Semi-permanent colouring is able to directly colour the hair without activating the oxidation process. They do not contain hydrogen peroxide and do not lighten the colour of the natural base, they can, so to speak, colour only by depositing colouring substances on the surface of the hair, and inside the cuticular layers.

Demipermanent colouring is a cross between direct semi-permanent and permanent colouring. It contains both direct colouring agents and bases and couplers, the typical colouring agents of oxidation colouring, however it has a lower quantity of hydrogen peroxide, generally no more than 7-8 volumes, compared to 20-40 volumes of a permanent colour. It therefore has no lightening power like permanent colouring and for this reason it is often also referred to as tone-on-tone colouring.

Metal salt based dyes are mainly used for covering the greying of white hair. Mostly used in the past and containing metal salts of lead (e.g. lead acetate), silver (silver nitrate), bismuth (bismuth citrate) etc. These colourings are said to be instantaneous if they cause the desired colour variation following a single application or progressive if the result is a consequence of several applications.

Colouring with dye plants envisages applying colouring agents typically of vegetable origin to the hair. In this context, one of the most widely used natural colouring agents is henna. This colouring agent is obtained from the leaves of the plant *Laswonia inermis.*

Henna is able to revive the natural colour of the hair and impart them a colouring that varies from warm red-orange to mahogany, depending on the starting colour of the treated hair. The colouring capacity of henna is mainly due to the presence of lawsone or 2-hydroxy-1,4-naphthoquinone.

Dye plants are commonly used in cosmetics to revive the streaks of the hair, especially in cases where white hair is absent or in any case sparse. In these conditions, plant-based colouring agents represent an interesting natural alternative to synthetic permanent colourings.

Typically, most of the dyes the colouring action of which is based on the use of dye plants are prepared extemporaneously, at the time of use, by mixing the dye plant (or the mixtures of dye plants) in powder form with hot water, until a mixture is obtained which is distributed on the hair. This mixture is removed from the hair after a suitable leave-on-time which varies according to the origin of the dye and the intensity of the colour to be obtained.

The mixture can also be left applied for a whole night and then rinsed off the following morning. Generally, the hair is tied and confined within a plastic cap, in order to reduce heat dispersion and not to dirty.

The colourings obtained by resorting to the use of dye plants are still widespread today although they provide a lower cosmetic result than the treatments that use synthetic colouring agents.

Sometimes direct colouring agents, such as sodium picramate, can be added to colourings with dye plants to obtain more vivid colourings.

The starting materials, used for the production of colourings based on dye plants, are generally leaves or other parts of the plant material, suitably chopped or pulverized. Finer raw materials generally have higher costs than raw materials with coarser dimensional characteristics.

However, the natural dyes currently on the market have a low resistance to washing and therefore a limited duration over time that does not meet the needs of a slice of consumers.

In an attempt to remedy these drawbacks, dyes have been formulated in which the dyeing component of natural origin is combined with natural substances, typically oils, which increase the adhesion of the natural colouring component to the hair keratin. However, these dyes have the drawback of making the hair excessively heavy and making it excessively greasy.

In an attempt to overcome this drawback, the consumer is led to increase the frequency of hair washing. However, a repeated and close washing action over time in turn reduces the shine and permanence of the natural colouring.

Currently, there is a growing demand that is not completely satisfied for new cosmetic compositions containing colouring substances of natural origin which are suitable for hair dyeing.

One of the objects of the invention therefore consists in providing an easy-to-use cosmetic composition for dyeing hair which is based on colouring substances of vegetable origin with colouring and cosmetic performances superior to those currently in use, also by resorting to the use of coarser powder mixtures.

A further object of the invention is to provide a dye which allows to easily disperse the dye powders and to easily and uniformly distribute the colouring components along the keratin fibres of the hair, providing uniform colouring.

### SUMMARY OF THE INVENTION

Within the technical field of the invention, the Applicant has found that by combining a dyeing component of vegetable origin with a specific dispersing/wetting substance, a uniform colouring is obtained along the entire shaft of the hair and a correlated appreciable aesthetic effect.

In view of the objects referred to above, the present invention provides, in accordance with a first aspect, a hair dye in the form of powder or granules comprising a cosmetic composition, for the extemporaneous colouring of hair, comprising a colouring component of vegetable origin based on one or more dye plants in powder and/or granules in combination with a dispersing component, characterised in that the dispersing component comprises a sucroester.

Advantageously, the powder based on one or more dye plants does not contain or may not contain colouring substances of synthetic origin.

Advantageously, the hair dye for hair colouring is presented as a non-aggregated powder, typically dry to the touch, a mixture of powders or a granular product.

According to the present invention, the hair dye does not contain an oil and/or an anionic surfactant. The hair dye of the invention is not in liquid form, for example of shampoo.

Typically, the hair dye in solid form of the invention is added with water preferably warm at the time of use, extemporaneously, to give a heterogeneous semi-solid slurry, poultice, cataplasm or mixture which is applied to the hair.

The hair dye in solid form is used for the extemporaneous colouring of the hair in a domestic environment, typically after adding water.

In the context of applications in the cosmetic or aesthetic field, the hair dye of the invention finds specific indication in hair dyeing.

In accordance with a second aspect, the present invention relates to the cosmetic use of the hair dye as defined previously comprising a colouring agent based on dye plants in combination with a sucroester to colour a hair and/or keratin structure of the human body, in particular hair, eyelashes and eyebrows.

The present disclosure also relates to a method for colouring a keratin structure or hair of the human body, in particular the hair, said method comprising the application on the keratin structure of a hair dye as previously defined comprising a colouring agent based on dye plants, in the form of powder and/or granules, in combination with a sucroester.

According to an embodiment, the combination of the vegetable colouring agent based on dye plant with sucrose esters and fatty acids is added with a rheological modifier and with water to form a poultice which is used to colour the hair.

In a third aspect, the invention relates to the use of a sucrose ester with a fatty acid or sucroester, as a dispersant or wetting agent of a solid colouring agent of vegetable origin based on one or more dye plants in powder and/or granules.

### Brief description of the figures

The characteristics and advantages of the present invention will become more evident from the attached drawing table in which:
Figure 1 illustrates hair strands used in the comparative test of Example 1;
Figure 2 illustrates three strands used in comparative example 1 in which strand 1 was treated with a composition based on sucroester and henna, strand two with Argan oil and henna, strand three only with henna. Strand 1 shows more vibrant colours, and better texture, softness and combability;
Figure 3 illustrates the results of Comparative Example 1 the day after the treatment on three pairs of strands;
Figure 4 illustrates the results of the in vivo treatment on hair with the composition based on sucroesters and henna according to Example 2.

### Detailed description of the invention

The present invention originates from having found that a hair dye is obtained by combining a vegetable dyeing component with a sucroester which can be easily applied to keratin structures, typically hair, to visibly improve the aesthetic appearance thereof and modify the original colouring thereof.

The combination of ingredients of the hair dye gives cosmetic performances superior to those of traditional dyeing products containing a dye plant powder. Said cosmetic performances include more vivid colours, a better workability of the preparation and a greater softness of the hair, obtained without the addition of oils.

In the hair dye of the invention, the vegetable dyeing or colouring component based on a dye plant is in powder and/or granules, for example obtained by grinding or mechanical treatment in order to reduce the size of a dye plant or portion thereof.

Advantageously, the hair dye based on dye plants is in the form of ground vegetable material, crushed pulverized, and is in the form of powder and/or granules.

In some embodiments, the colouring component based on a dye plant has a fine grain size and is in powder form.

In the present context, the term powder means an average particle size ranging from 10 to 500 microns.

In some embodiments the hair dye further comprises the vegetable colouring component based on a dye plant in the form of granules.

In the present context, the term granules means an average particle size > 500 microns, for example from 600 to 2000 microns, from 800 to 1500 microns, from 1000 to 1200 microns.

In accordance with some embodiments, it has been observed that, by using the dispersing agent based on sucroester, the vegetable dyeing component can contain the colouring component based on a dye plant in granules or coarse powders having a size greater than 600 microns for example from 600 to 2000 microns, for example in a quantity from 0.5 to 15% by weight with respect to the total weight of the dyeing component, without significantly changing the aesthetic or colouring effect.

It has been surprisingly observed that the addition of amphiphilic substances belonging to the family of sucroesters allows to obtain a colouring and/or a cosmetic effect superior to those of dyes containing dyeing powders with very fine particle sizes, for example ranging from 10 to 500 microns added with an oil.

This effect allows the use of a lower quantity of colouring powder than those of dyes with finer powders to dye the hair and greater safety of use.

According to some embodiments, the dyeing component of the composition has a water content ranging from 5 to 20%, from 8 to 15% by weight, relative to the total weight of the dyeing powders of the composition.

The vegetable dyeing component comes from any plant or portion of a plant endowed with dyeing properties. Advantageously, the dye plant used for the formulation of the composition is chopped or ground, in the form of preferably coarse or semi-fine powder.

For the purposes of the applications of the present invention, a suitable dye plant powder can be obtained by mechanical grinding or reduction of roots, leaves, fruits, berries or flowers of the plant or from two or more of these parts of the plant.

In particular, the dyeing component in powder form can be obtained by grinding a portion coming from the root system, from the aerial portion, for example stem or leaves, or from the fruit of a dye plant.

Suitable dye plants comprise henna, typically *Lawsonia inermis,* indigo (*Indigofera tinctoria*)*,* cassia (*Cassia spp. For example, C. italica* also called *Cassia obovata*)*,* rhubarb (*Rheum palmatum*)*,* walnut (*Juglans Regia*)*.*

In the context of the invention, the use of indigo, henna and cassia is preferred among dye plants.

Indigo (*Indigofera tinctoria*) is a plant of Indian origin, whose leaves contain various substances such as flavonoids, terpenoids, alkaloids and tannins.

The substance responsible for the colouring is actually *the indican,* a glycoside that hydrolyzes by releasing *indoxile* (and glucose).The *indoxile* by the action of atmospheric oxygen turns into *indigotine* also called *indigo.*

*Cassia italica* also called *Cassia obovata* is also known as neutral, or neutral henna, as it does not have a particular colouring capacity. It is also used as a diluent and base for lighter colourings, it also has substantiating properties.

Together with the colouring properties, these preparations are able to improve the structural characteristics of the hair, which become less fragile as the treatments carry on.

In accordance with some embodiments, the dyeing component of the composition comprises a combination of powders of two or more dye plants in order to provide a wide range of colourings and a variety of shades and streaks.

According to some embodiments, the composition of the invention contains the dyeing component, in the form of powder and/or granules, in a quantity ranging from 1 to 99.5%, from 20 to 90%, from 40 to 80% by weight with respect to the total weight of the composition.

In accordance with some embodiments, the dyeing component is selected from henna, a plant that produces indigo and mixtures thereof, preferably in the form of powder and/or granules as previously described.

By way of example, the powder dyeing component can be obtained by subjecting a dye plant or the dyeing portion thereof to grinding. The obtained powders are sieved with mechanical sieves, selecting the powder fraction with dimensions ranging from 10 to 500 µm.

Suitable mechanical sieves are commercially available and are generally indicated with the "mesh" unit: the powder fraction with particle size equal to or less than 500 µm is collected by sieving the powders through a commercial 35 mesh sieve in accordance with ASTM Standard. This step helps calibrate the powders and remove larger aggregates.

Advantageously, the hair dye of the invention is in the powdery and/or granule form.

For the purposes of the present invention, granules or granulates refer to mixtures of solid particles which by virtue of their shape, size (dimensional distribution) have a lower dustiness and advantageous flowing properties which are far higher than those of impalpable powders that are typically little flowing. The flowing properties of a powder or granular material can be determined by using suitable devices and methods typically used in the pharmaceutical technique (e.g. Flodex: Powder Flowability Test Apparatus Hanson Research/ Teledyne).Flowing property refers to the property of a powder to flow uniformly under the action of gravity or other forces applied thereto. The flowing properties of a powder can be increased through a process known as granulation, which can be done using wet or dry techniques, with the use of special equipment (for example fluid beds, fast granulators, etc.).Wet granulation typically provides for the use of a binder, generally a polymer, natural or not. For the purposes of the present invention and with the purpose of transforming the raw material from powdery to granular, a multiplicity of synthetic and natural ingredients and combinations thereof, such as for example monosaccharides (such as glucose), disaccharides (such as sucrose), trisaccharides, oligosaccharides, polysaccharides such as dextrins and maltodextrins, cellulose and derivatives thereof (such as hydroxyethylcellulose, hydroxypropyl-methylcellulose), polyols, 1-vinyl-2-pyrrolidone polymers (such as for example polyvinylpyrrolidone) and derivatives thereof can be used as binders; but not limited to them. The granulate differs from the starting powder as it consists of more or less cohesive aggregates of particles. The hair dye of the invention comprises at least one sucroester as a dispersant or dispersing agent.

Within the scope of the invention the term sucroester means sucrose esters with both saturated and unsaturated or polyunsaturated fatty acids. Typically, the sucroester is a non-ionic surfactant. Advantageously, the hair dye of the invention does not contain an anionic surfactant since its presence would reduce the dispersing effect of the sucroester and make the hair colouring less uniform after application.

According to some embodiments the fatty acids are saturated fatty acids with a number of carbon atoms ranging from 4 to 32, preferably selected from lauric acid, myristic acid, palmitic acid, stearic acid, arachidic acid and behenic acid, or they are unsaturated fatty acids with a number of carbon atoms from 14 to 24 and preferably are palmitoleic acid, oleic acid, erucic acid or polyunsaturated such as linoleic, linolenic acid and others.

Sucroesters originate from the esterification of sucrose with single fatty acids or mixtures thereof or from esters thereof in the case of transesterification.

As regards the degree of esterification, for the purposes of the present invention the following can be used: monoesters, diesters, triesters or higher esters and combinations thereof.

Typically, the sucroesters used herein, being amphiphilic, can be classified on the basis of the hydrophilic/lipophilic balance.

This hydrophilic/lipophilic balance, whose acronym is HLB, allows to assign a score to each raw material, ranging between 0 and 20, where zero coincides with a totally lipophilic substance, while 20 coincides with total hydrophilicity.

In a preferable embodiment of the invention, said HLB of the sucrose esters of the fatty acids ranges between 4 and 9.

In a still more preferable embodiment of the invention, the HLB of the sucrose esters of fatty acids ranges between 5-7.

In a particular embodiment of the invention, HLB is approximately 6 and originates from the use of sucroesters in which the fatty acids are a mixture of stearic and palmitic acid in a ratio of about 60-80/40-20, in which the percentage of monoester is about 30%, of the diester about 50% and about 20% of higher esters. Examples of suitable sucroesters are: Sucrose laurate; Sucrose myristate, Sucrose palmitate, Sucrose stearate, Sucrose behenate, Sucrose oleate, Sucrose ricinoleate, Sucrose dilaurate, Sucrose distearate, Sucrose tetraisostearate, Sucrose tribehenate, Sucrose trilaurate, Sucrose tristearate, Sucrose palmitate/Stearate, Sucrose cocoate, Sucrose cottonseedate, Sucrose palmate and mixtures thereof.

Typically, the sucroesters can be prepared from sucrose and methyl and ethyl esters of fatty acids or by extraction from sucroglycerides using organic solvents such as for example dimethylformamide, formamide, dimethyl sulfoxide, ethyl acetate, isopropanol, propylene glycol, isobutanol and methyl ethyl ketone.

In one embodiment of the invention the composition described herein further comprises a polymer or oligomers of a protein nature, such as for example sericin or hydrolyzed keratin.

In a preferable embodiment of the invention said rheological modifier is tamarind rubber.

In a still more preferable embodiment of the invention said rheological modifier is the tamarind polysaccharide (*Tamarindus indica* L.) known as galactoxyloglucan. For the purposes of the present invention, tamarind gum means the products of the processing of tamarind seeds, *Tamarindus indica* Linné, an evergreen plant belonging to the subfamily of *Caesalpinioideae,* family of legumes (Fabaceae), containing a branched polysaccharide, a galactoxyloglucan, composed of glucose, xylose and galactose.

For the purposes of the present invention, tamarind polysaccharides refer to raw materials obtained from the processing of the endosperm of the tamarind seed containing a high polysaccharide content, indicatively higher than 70%.

The composition of the invention is intended for external use, in particular for colouring the hair of a human being.

The composition of the invention may further comprise a cosmetically acceptable vehicle.

In the context of the present invention, the term "vehicle" refers to an excipient, vehicle, diluent, or adjuvant which may be present in the composition of the invention.

Typically, the composition described herein can be used extemporaneously, at the time of use, by mixing it with preferably hot water, for example between 35 and 70°C, until a mixture is obtained which is then distributed on the hair. The water is often brought to a boil and then the preparation applied to the hair when the temperature has dropped to a temperature that is not perceived as too hot when applied to the hair. This mixture is removed from the hair after a suitable leave-on-time typically from 30 minutes to 2 hours, depending on the intensity of the colouring to be obtained.

The mixture can also be left applied for a whole night and then rinsed off the following morning. Typically, during colouring, the hair is tied and confined within a plastic cap, in order to reduce heat dispersion and not to dirty.

The present invention will now be described with reference to the following examples which are provided for illustrative purposes.

### EXAMPLE No. 1

The tests aimed at proving the technical effect of the composition of the invention were carried out first on a strand of hair and then on hair of a female individual.

### a. Materials

100% bleached white hair strands; 3 ± 0.2 grams
4 auburn strands; 3 ± 0.2 grams
Shampoo: Internal basic formula

### b. Pre-treatment of strands

Before use, the strands were rinsed with warm water, then washed with 0.25g of shampoo, then thoroughly rinsed with warm water and dried for 5 minutes with a hairdryer. The hair was then untangled with 10 brush strokes.

### c. Preparation of colouring mixtures

3% of the following ingredients have been added to 97 grams of LAWSONIA INERMIS LEAF EXTRACT:
1. SUCROSE PALMITATE (HLB ≈16, HIGH MONOESTER ≥ 60%)
2. SUCROSE STEARATE (HLB ≈ 15, HIGH MONOESTER≥ 60%)
3. SUCROSE DISTEARATE (HLB ≈ 6, LOW MONESTER ≤ 30)
4. SUCROSE POLYSTEARATE (HLB ≈ 1, LOW MONOESTER ≤30%)
5. SUCROSE PALMITATE (HLB ≈15, HIGH MONOESTER ≥ 60%)
6. SUCROSE PALMITATE (HLB ≈16, HIGH MONOESTER ≥ 60%)

As control was used:
7. ARGAN OIL (Organic Virgin Deodorised Argan Oil, Olvea Group)

One part of the mixture was diluted with 3 parts of very hot water, about 80°C at the end of homogenization in a bowl with a brush.

A weighed quantity of the mixture thus obtained was applied to the strand (previously wet with water) by brush, the strands were then covered with a polyethylene-based food film and left on for 30 and 60 minutes.

After the planned leave-on-time, the strands were rinsed with warm water.

The strands were then dried and combed in the manner described above.

The treated and dried strands were then subjected to the assessment of a small panel of 6 women, according to a blind mode study where the panelists were not aware of the treatment applied to the strand.

This procedure was used in order to obtain objective results not influenced by external aspects.

The parameters assessed were:
Colour rendering (colour intensity)
Softness
Brightness/shine
Texture
Coverage of white hair (in the case of 100% bleached white hair)

The results obtained highlight how the application of sucroesters improves the cosmetic performance with respect to mixtures with and without Argan oil.

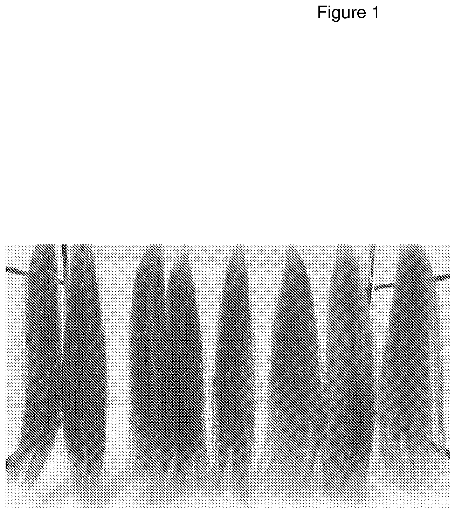

In particular, Figure 1 shows six strands, four of which are treated from left to right as follows: strands 1, 2, 5 and 6 with different esters of sucrose (3%) and henna (97%); third and fourth strands with argan oil (3%) with henna (97%); 7 and 8 only henna (100%).

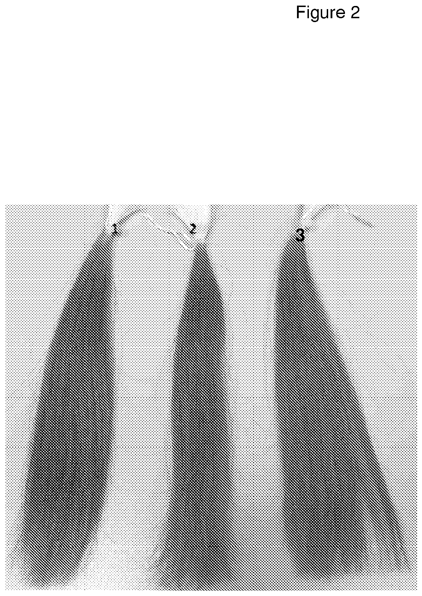

Figure 2 shows images comparing three strands treated, respectively, with compositions of sucroester and henna (1), argan oil and henna (2) and only henna (3) in the percentages reported above.

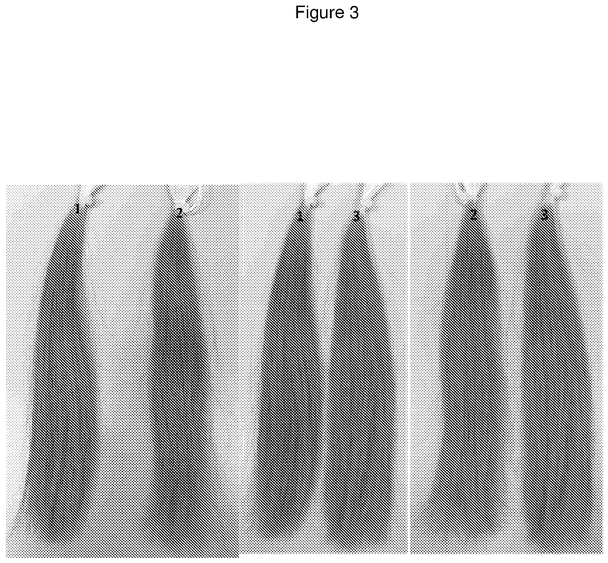

As can also be seen from Figure 3, the strands treated with sucroester show more vibrant colours which can be better appreciated by direct visual observation. The assessment of the texture, softness, combability and appearance by the panelists returned unequivocal data that hair treated with sucroesters perform better.

The sensory assessment of the strands was repeated the next day. The results are illustrated in Figure 3.

### Test on hair of individuals

Compositions of sucroester and Henna having a formulation according to Example 2 were tested on hair of subjects.

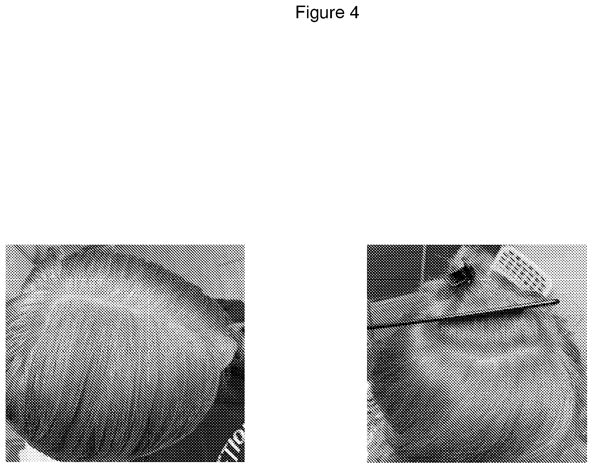

The results obtained on the combinations of the compositions of Examples 3, 5 and 7 were applied on female subjects and confirmed the observations on the strand. In Figure 4 the left image refers to the hair of a female individual before treatment, the right one after treatment with the formulation of Example 2.

The results of the in vivo tests confirmed the results obtained on the strand.

The in vivo tests were extended to compositions containing dye plants other than Henna such as those of Example 5 containing Indigo and that of Example 7 containing Cassia.

The tests on the hair of volunteers were also carried out using a benchmark obtained from the market, in the application mode of the half head.

In the following examples the quantities of the ingredients are expressed in % in w/w.

### EXAMPLE No. 2

### Copper red colouring mixture

| Component (INCI Name) | %w/w |
|---|---|
| LAWSONIA INERMIS LEAF EXTRACT | 96 - 99.5 |
| SUCROSE DISTEARATE | 0.5 - 4 |

### EXAMPLE No. 3

### Copper red colouring mixture

| **Component (INCI Name)** | **%w/w** |
|---|---|
| TAMARINDUS INDICA SEED POLYSACCHARIDE | 0.05 - 1 |
| LAWSONIA INERMIS LEAF EXTRACT | 90 - 99 |
| SERICIN | 0.01 - 1 |
| SUCROSE DISTEARATE | 0.5 - 4 |

### EXAMPLE No. 4

### Auburn 1 colouring mixture

| **Component (INCI Name)** | **%w/w** |
|---|---|
| CYAMOPSIS TETRAGONOLOBA GUM | 0.1 - 1 |
| LAWSONIA INERMIS LEAF EXTRACT | 60 - 90 |
| INDIGOFERA TINCTORIA LEAF POWDER | 10 - 40 |
| SUCROSE STEARATE (LOW MONOESTER ≤30%) | 0.5 - 4 |

### EXAMPLE No. 5

### Auburn 1 colouring mixture

| **Component (INCI Name)** | **%w/w** |
|---|---|
| TAMARINDUS INDICA SEED POLYSACCHARIDE | 0.05 - 1 |
| LAWSONIA INERMIS LEAF EXTRACT | 60 - 90 |
| INDIGOFERA TINCTORIA LEAF POWDER | 10 - 40 |
| SERICIN | 0.01 - 1 |
| SUCROSE DISTEARATE | 0.5 - 4 |

### EXAMPLE No. 6

### Auburn 2 colouring mixture

| **Component (INCI Name)** | **%w/w** |
|---|---|
| LAWSONIA INERMIS LEAF EXTRACT | 20 - 60 |
| INDIGOFERA TINCTORIA LEAF POWDER | 5 - 50 |
| CASSIA ITALICA LEAF EXTRACT | 20 - 60 |
| SUCROSE STEARATE (HIGH MONOESTER ≥ 60%) | 0.5 - 4 |

### EXAMPLE No. 7

### Auburn 2 colouring mixture

| **Component (INCI Name)** | **%w/w** |
|---|---|
| TAMARINDUS INDICA SEED POLYSACCHARIDE | 0.05 - 1 |
| LAWSONIA INERMIS LEAF EXTRACT | 20 - 60 |
| INDIGOFERA TINCTORIA LEAF POWDER | 5 - 50 |
| CASSIA ITALICA LEAF EXTRACT | 20 - 60 |
| SERICIN | 0.01 - 1 |
| SUCROSE DISTEARATE | 0.5 - 4 |

### EXAMPLE No. 8

### Auburn 2 colouring mixture

| **Component (INCI Name)** | **%w/w** |
|---|---|
| TAMARINDUS INDICA SEED POLYSACCHARIDE | 0.05 - 1 |
| LAWSONIA INERMIS LEAF EXTRACT | 20 - 60 |
| INDIGOFERA TINCTORIA LEAF POWDER | 5 - 50 |
| CASSIA ITALICA LEAF EXTRACT | 20 - 60 |
| SERICIN | 0.01 - 1 |
| SUCROSE STEARATE (HIGH MONOESTER > 60%) | 0.5 - 4 |

### EXAMPLE No. 9

### Copper red colouring mixture

| Component (INCI Name) | %w/w |
|---|---|
| LAWSONIA INERMIS LEAF EXTRACT | 96 - 99.5 |
| SUCROSE STEARATE (MEDIUM MONOESTER: 60 >MONOESTER > 30 %) | 0.5 - 4 |

### EXAMPLE No. 10

Particle size analysis by vibrating screen (Octagon 200, Endecotts Limited, London UK) of a sample weighing 40.04 of natural henna colouring agent used in the preparation (mixing of coarse henna powder with sucroester at room temperature with mixer) of the composition of the Example 2.

### Natural copper colouring

| sieve (mesh) | micron | ASTM E 11-70 | tare | gross | net | % |
|---|---|---|---|---|---|---|
| plate | <106 | | 242.84 | 247 | 4.16 | 10.38961 |
| 140 | 106 | <150 | 267.81 | 273.58 | 5.77 | 14.41059 |
| 100 | 150 | <212 | 269.54 | 278.5 | 8.96 | 22.37762 |
| 70 | 212 | <250 | 295.7 | 301.53 | 5.83 | 14.56044 |
| 60 | 250 | <300 | 287.79 | 291.9 | 4.11 | 10.26474 |
| 50 | 300 | <425 | 294.99 | 302.95 | 7.96 | 19.88012 |
| 40 | 425 | <600 | 308.51 | 309.8 | 1.29 | 3.221778 |
| 30 | 600 | <850 | 320.98 | 321.4 | 0.42 | 1.048951 |
| 20 | 850 | <1180 | 360.28 | 361.63 | 1.35 | 3.371628 |
| 16 | 1180 | >1180 | 357.3 | 357.43 | 0.13 | 0.324675 |
| | | | | | 39.98 | 99.85015 |

| Weight sample (g) | 40.04 |
|---|---|
| Amplitude | 5 |
| time | 10 |

### Natural blond colouring

| sieve (mesh) | micron | ASTM E 11-70 | tare | gross | net | % |
|---|---|---|---|---|---|---|
| plate | <106 | | 242.84 | 248.68 | 5.84 | 14.63292 |
| 140 | 106 | <150 | 267.81 | 277.28 | 9.47 | 23.72839 |
| 100 | 150 | <212 | 269.54 | 275.07 | 5.53 | 13.85618 |
| 70 | 212 | <250 | 295.7 | 298.83 | 3.13 | 7.842646 |
| 60 | 250 | <300 | 287.79 | 288.64 | 0.85 | 2.129792 |
| 50 | 300 | <425 | 294.99 | 303.98 | 8.99 | 22.52568 |
| 40 | 425 | <600 | 308.51 | 310.33 | 1.82 | 4.560261 |
| 30 | 600 | <850 | 320.98 | 321.44 | 0.46 | 1.152593 |
| 20 | 850 | <1180 | 360.28 | 363.85 | 3.57 | 8.945127 |
| 16 | 1180 | >1180 | 357.3 | 357.43 | 0.13 | 0.325733 |
| | | | | | 39.79 | 99.69932 |

| Sample weight (g) | 39.91 |
|---|---|
| Amplitude | 5 |
| time | 10 |

### Dark auburn colouring

| sieve (mesh) | micron | ASTM E 11-70 | tare | gross | net | % |
|---|---|---|---|---|---|---|
| plate | <106 | | 242.84 | 245.08 | 2.24 | 5.591613 |
| 140 | 106 | <150 | 267.81 | 273.44 | 5.63 | 14.05392 |
| 100 | 150 | <212 | 269.54 | 276.23 | 6.69 | 16.69995 |
| 70 | 212 | <250 | 295.7 | 300.94 | 5.24 | 13.08038 |
| 60 | 250 | <300 | 287.79 | 290.93 | 3.14 | 7.838243 |
| 50 | 300 | <425 | 294.99 | 309.38 | 14.39 | 35.92112 |
| 40 | 425 | <600 | 308.51 | 310.16 | 1.65 | 4.118822 |
| 30 | 600 | <850 | 320.98 | 321.16 | 0.18 | 0.449326 |
| 20 | 850 | <1180 | 360.28 | 361.2 | 0.92 | 2.296555 |
| 16 | 1180 | >1180 | 357.3 | 357.37 | 0.07 | 0.174738 |
| | | | | | 40.15 | 100.2247 |

| Sample weight (g) | 40.06 |
|---|---|
| Amplitude | 5 |
| time | 10 |

## Claims

1. Hair dye in the form of powder or granules comprising a cosmetic composition, for the extemporaneous colouring of hair, comprising a colouring component of vegetable origin based on one or more dye plants in powder and/or granules in combination with a dispersing component, **characterised in that** the dispersing component comprises a sucroester.

2. Hair dye according to claim 1, wherein said sucroester is a sucrose ester with a saturated fatty acid having 2 to 32 carbon atoms or a sucrose ester with a monounsaturated fatty acid having 14 to 24 carbon atoms or a sucrose ester with a polyunsaturated fatty acid having 18 to 22 carbon atoms.

3. Hair dye according to claim 1 or 2, wherein the dye plant is selected from henna, indigo, cassia, rhubarb, walnut, and mixtures thereof, and is preferably selected from indigo, henna, cassia, and mixtures thereof.

4. Hair dye according to any one of claims 1-3, wherein the dye plant in powder and/or granules is in an amount ranging from 1 to 99.5%, preferably from 20 to 90%, more preferably from 40 to 80% by weight in relation to the total weight of the composition.

5. Hair dye according to any one of claims 1-4, wherein the sucroester is in an amount ranging from 1% to 50% by weight in relation to the total weight of the composition.

6. Hair dye according to any one of claims 1-5, wherein the colouring component of vegetable origin is henna.

7. Hair dye according to any one of claims 1-6, further comprising a polymer or oligomers of protein nature, preferably sericin or the hydrolysed keratin.

8. Hair dye according to any one of claims 1-7, further comprising a rheological modifier, preferably tamarind gum or galactoxyloglucan.

9. Cosmetic use of a hair dye according to any one of claims 1-8, for the extemporaneous dyeing of a keratin structure, preferably hair, eyelashes, or eyebrows of a human being.

10. Use of a sucrose ester with a fatty acid or sucroester as a dispersant or wetting agent of a hair colouring agent in solid form of vegetable origin based on one or more dye plants in powder and/or granules.

## Patentansprüche

1. Haarfärbemittel in Form von Pulver oder Granulat, das eine kosmetische Zusammensetzung für das spontane Färben von Haaren aufweist, die eine färbende Komponente pflanzlichen Ursprungs auf der Basis einer oder mehrerer Färbepflanzen in Pulver- und/oder Granulatform in Kombination mit einer dispergierenden Komponente aufweist, **dadurch gekennzeichnet, dass** die dispergierende Komponente einen Zuckerester aufweist.

2. Haarfärbemittel nach Anspruch 1, wobei der Zuckerester ein Zuckerester mit einer gesättigten Fettsäure mit 2 bis 32 Kohlenstoffatomen oder ein Zuckerester mit einer einfach ungesättigten Fettsäure mit 14 bis 24 Kohlenstoffatomen oder ein Zuckerester mit einer mehrfach ungesättigten Fettsäure mit 18 bis 22 Kohlenstoffatomen ist.

3. Haarfärbemittel nach Anspruch 1 oder 2, wobei die Färbepflanze ausgewählt ist aus Henna, Indigo, Cassia, Rhabarber, Walnuss und Mischungen davon, und vorzugsweise ausgewählt ist aus Indigo, Henna, Cassia und Mischungen davon.

4. Haarfärbemittel nach einem der Ansprüche 1 bis 3, wobei die Färbepflanze in Form von Pulver und/oder Granulat in einer Menge von 1 bis 99,5 %, vorzugsweise von 20 bis 90 %, besonders bevorzugt von 40 bis 80 Gew.-%, bezogen auf das Gesamtgewicht der Zusammensetzung, vorliegt.

5. Haarfärbemittel nach einem der Ansprüche 1 bis 4, wobei der Zuckerester in einer Menge von 1 bis 50 Gew.-%, bezogen auf das Gesamtgewicht der Zusammensetzung, vorliegt.

6. Haarfärbemittel nach einem der Ansprüche 1 bis 5, wobei die färbende Komponente pflanzlichen Ursprungs Henna ist.

7. Haarfärbemittel nach einem der Ansprüche 1 bis 6, ferner aufweisend ein Polymer oder Oligomere von Protein-Natur, vorzugsweise Sericin oder das hydrolysierte Keratin.

8. Haarfärbemittel nach einem der Ansprüche 1 bis 7, ferner aufweisend einen rheologischen Modifikator, vorzugsweise Tamarindengummi oder Galactoxyloglucan.

9. Kosmetische Verwendung eines Haarfärbemittels nach einem der Ansprüche 1 bis 8 zum spontanen Färben einer Keratinstruktur, vorzugsweise von Haaren, Wimpern oder Augenbrauen eines Menschen.

10. Verwendung eines Zuckeresters mit einer Fettsäure oder eines Zuckeresters als Dispergiermittel oder Netzmittel eines Haarfärbemittels in fester Form pflanzlichen Ursprungs auf der Basis einer oder mehrerer Färbepflanzen in Pulver- und/oder Granulatform.

## Revendications

1. Colorant capillaire sous forme de poudre ou de granulés comprenant une composition cosmétique, pour la coloration extemporanée des cheveux, comprenant un composant colorant d'origine végétale à base d'une ou plusieurs plantes colorantes en poudre et/ou en granulés en combinaison avec un composant dispersant, **caractérisé en ce que** le composant dispersant comprend un sucroester.

2. Colorant capillaire selon la revendication 1, dans lequel ledit sucroester est un ester de sucrose avec un acide gras saturé ayant de 2 à 32 atomes de carbone ou un ester de sucrose avec un acide gras monoinsaturé ayant de 14 à 24 atomes de carbone ou un ester de sucrose avec un acide gras polyinsaturé ayant de 18 à 22 atomes de carbone.

3. Colorant capillaire selon la revendication 1 ou 2, dans lequel la plante colorante est choisie parmi le henné, l'indigo, la casse, la rhubarbe, le noyer et leurs mélanges, et est de préférence choisie parmi l'indigo, le henné, la casse et leurs mélanges.

4. Colorant capillaire selon l'une quelconque des revendications 1 à 3, dans lequel la plante colorante en poudre et/ou en granulés est en quantité allant de 1 à 99,5 %, de préférence de 20 à 90 %, plus préférentiellement de 40 à 80 % en poids par rapport au poids total de la composition.

5. Colorant capillaire selon l'une quelconque des revendications 1 à 4, dans lequel le sucroester est en quantité allant de 1 % à 50 % en poids par rapport au poids total de la composition.

6. Colorant capillaire selon l'une quelconque des revendications 1 à 5, dans lequel le composant colorant d'origine végétale est le henné.

7. Colorant capillaire selon l'une quelconque des revendications 1 à 6, comprenant en outre un polymère ou des oligomères de nature protéique, de préférence de la séricine ou de la kératine hydrolysée.

8. Colorant capillaire selon l'une quelconque des revendications 1 à 7, comprenant en outre un modificateur rhéologique, de préférence de la gomme de tamarin ou du galactoxyloglucan.

9. Utilisation cosmétique d'un colorant capillaire selon l'une quelconque des revendications 1 à 8, pour la coloration extemporanée d'une structure kératinique, de préférence les cheveux, les cils ou les sourcils d'un être humain.

10. Utilisation d'un ester de sucrose avec un acide gras ou un sucroester comme dispersant ou agent mouillant d'un colorant capillaire sous forme solide d'origine végétale à base d'une ou plusieurs plantes colorantes en poudre et/ou en granulés.
